# EUROPÄISCHE PATENTANMELDUNG

(11) **EP 3 299 457 A1**
(43) Veröffentlichungstag der Anmeldung: **28.03.2018**
(21) Anmeldenummer: 17186570.2
(22) Anmeldetag: 17.08.2017
(51) Int. Cl.: C12N 9/18, C12N 9/20, C11D 3/386

(54) **NEUE LIPASE**

(30) Priorität: 26.09.2016 DE 102016218443
(71) Anmelder: Henkel AG & Co. KGaA, 40589 Düsseldorf (DE)
(72) Erfinder: HERBST, Daniela, 40237 Düsseldorf (DE); MUßMANN, Nina, 47877 Willich (DE); WIELAND, Susanne, 41541 Zons/Dormagen (DE); ELLEUCHE, Skander, 21129 Hamburg (DE); BUSCH, Philip, 21073 Hamburg (DE); LORENZ, Ute, 25474 Ellerbek (DE); ANTRANIKIAN, Garabed, 21218 Seevetal-Hittfeld (DE)

(57) **Zusammenfassung**

Die Erfindung betrifft Lipasen umfassend eine Aminosäuresequenz, die mindestens 70 % Sequenzidentität mit der in SEQ ID NO:1 angegebenen Aminosäuresequenz über deren Gesamtlänge aufweist sowie deren Herstellung und Verwendung. Derartige Lipasen zeigen eine gute Reinigungsleistung.

## Beschreibung

Die Erfindung liegt auf dem Gebiet der Enzymtechnologie. Die Erfindung betrifft insbesondere Lipasen, die insbesondere im Hinblick auf den Einsatz in Wasch- und Reinigungsmitteln verwendet werden können, sowie deren Herstellung, deren Aminosäuresequenz, alle hinreichend ähnlichen Lipasen und für sie codierende Nukleinsäuren. Die Erfindung betrifft ferner Verfahren zu deren Verwendung und die Verwendung dieser Lipasen sowie sie enthaltende Mittel, insbesondere Wasch- und Reinigungsmittel.

Lipasen gehören zu den technisch bedeutendsten Enzymen überhaupt. Ihr Einsatz für Wasch- und Reinigungsmittel ist industriell etabliert und sie sind in praktisch allen modernen, leistungsfähigen Wasch- und Reinigungsmitteln enthalten. Eine Lipase ist ein Enzym, das die Hydrolyse von Esterbindungen in Lipid-Substraten, insbesondere in Fetten und Ölen, katalysiert. Lipasen stellen daher eine Gruppe der Esterasen dar. Lipasen sind im Allgemeinen vielseitige Enzyme, die eine Vielzahl an Substraten akzeptieren, beispielsweise aliphatische, alizyklische, bizyklische und aromatische Ester, Thioester und aktivierte Amine. Lipasen wirken gegen Fettrückstände in der Wäsche und katalysieren deren Hydrolyse (Lipolyse). Lipasen mit breiten Substratspektren werden insbesondere dort verwendet, wo inhomogene Rohstoffe oder Substratgemische umgesetzt werden müssen, also beispielsweise in Wasch- und Reinigungsmitteln, da Verschmutzungen aus unterschiedlich aufgebauten Fetten und Ölen bestehen können. Die in den aus dem Stand der Technik bekannten Wasch-oder Reinigungsmitteln eingesetzten Lipasen sind üblicherweise mikrobiellen Ursprungs und stammen in der Regel aus Bakterien oder Pilzen, beispielsweise der Gattungen *Thermomyces, Bacillus, Pseudomonas, Acinetobacter, Micrococcus, Humicola, Trichoderma* oder *Trichosporon.* Lipasen werden üblicherweise nach an sich bekannten biotechnologischen Verfahren durch geeignete Mikroorganismen produziert, beispielsweise durch transgene Expressionswirte der Gattungen Bacillus oder durch filamentöse Pilze.

In der deutschen Patentanmeldung DE 102012224038 A1 und der internationalen Patentanmeldung WO 2014152674 ist beispielsweise eine für Wasch- und Reinigungsmittel vorgesehene Lipase aus *Thermomyces lanuginosus* offenbart. Generell sind nur ausgewählte Lipasen für den Einsatz in flüssigen Tensidzubereitungen überhaupt geeignet. Viele Lipasen zeigen in derartigen Zubereitungen keine ausreichende katalytische Leistung oder Stabilität.

Daher besteht ein Bedarf an Lipase-Varianten, die Aktivität in Reinigungszusammensetzungen besitzen und veränderte biochemische Eigenschaften besitzen und dadurch eine verbesserte Leistung in derartigen Anwendungen bereitstellen.

Überraschenderweise wurde jetzt festgestellt, dass ein als Lipase annotiertes Enzym aus *Alicyclobacillus acidocaldarius* mit der Aminosäuresequenz gemäß SEQ ID NO:1 oder eine hierzu hinreichend ähnliche Lipase (bezogen auf die Sequenzidentität), besonders für den Einsatz in Wasch- oder Reinigungsmitteln geeignet ist, da sie in Reinigungszusammensetzungen stabil ist und ein breites Spektrum an Lipid-Substraten unter Standard-Waschbedingungen hydrolysiert.

Gegenstand der Erfindung ist daher in einem ersten Aspekt eine Lipase umfassend eine Aminosäuresequenz, die mindestens 70 % Sequenzidentität mit der in SEQ ID NO:1 angegebenen Aminosäuresequenz über deren Gesamtlänge aufweist.

Ein weiterer Gegenstand der Erfindung ist ein Verfahren zur Herstellung einer Lipase umfassend das Bereitstellen einer Ausgangslipase, die mindestens 70 % Sequenzidentität zu der in SEQ ID NO:1 angegebenen Aminosäuresequenz über deren Gesamtlänge aufweist.

Eine Lipase im Sinne der vorliegenden Patentanmeldung umfasst daher sowohl die Lipase als solche als auch eine mit einem erfindungsgemäßen Verfahren hergestellte Lipase. Alle Ausführungen zur Lipase beziehen sich daher sowohl auf die Lipase als Stoff als auch auf die entsprechenden Verfahren, insbesondere Herstellungsverfahren der Lipase, und die damit hergestellten Lipasen.

Weitere Erfindungsgegenstände sind die die erfindungsgemäßen Lipasen kodierenden Nukleinsäuren, erfindungsgemäße Lipasen oder Nukleinsäuren, die diese kodieren, enthaltende nicht menschliche Wirtszellen sowie erfindungsgemäße Lipasen umfassende Mittel, insbesondere Wasch- und Reinigungsmittel, Wasch- und Reinigungsverfahren in denen die erfindungsgemäßen Lipasen eingesetzt werden, und Verwendungen der erfindungsgemäßen Lipasen. Eine die Aminosäuresequenz gemäß SEQ ID NO:1 kodierende Nukleotidsequenz ist in SEQ ID NO:2 angegeben.

Die vorliegende Erfindung basiert auf der überraschenden Erkenntnis, dass eine erfindungsgemäße Lipase aus *Alicyclobacillus acidocaldarius,* die eine zu der in SEQ ID NO:1 angegebenen Aminosäuresequenz zu mindestens 70% identische Aminosäuresequenz umfasst, die Hydrolyse eines breiten Spektrums an Lipid-Substraten unter Standard-Waschbedingungen bewirkt. Das ist insbesondere insoweit überraschend, als dass bisher keine Lipasen mit vergleichbarer Sequenzhomologie für die Verwendung in Reinigungsmitteln beschrieben wurden.

Die erfindungsgemäßen Lipasen verfügen über eine hohe Stabilität in Wasch- oder Reinigungsmitteln, beispielsweise gegenüber Tensiden und/oder Bleichmitteln und/oder gegenüber Temperatureinflüssen, und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse. In besonders bevorzugten Ausführungsformen der Erfindung werden folglich leistungsverbesserte Lipase-Varianten bereitgestellt. Solche vorteilhaften Ausführungsformen erfindungsgemäßer Lipasen ermöglichen folglich verbesserte Waschergebnisse an lipidhaltigen Anschmutzungen in einem weiten Temperaturbereich.

Eine erfindungsgemäße Lipase weist eine enzymatische Aktivität auf, das heißt, sie ist zur Hydrolyse von Fetten und Ölen befähigt, insbesondere in einem Wasch- oder Reinigungsmittel. Eine erfindungsgemäße Lipase ist daher ein Enzym, welches die Hydrolyse von Esterbindungen in Lipid-Substraten katalysiert und dadurch in der Lage ist, Fette oder Öle zu spalten. Ferner handelt es sich bei einer erfindungsgemäßen Lipase vorzugsweise um eine reife (mature) Lipase, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung umfasst die Lipase eine Aminosäuresequenz, die zu der in SEQ ID NO:1 angegebenen Aminosäuresequenz über deren Gesamtlänge zu mindestens 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,8%, 99,0%, 99,2%, 99,4%, 99,6% oder 99,8% identisch ist. In weiteren Ausführungsformen ist die erfindungsgemäße Lipase zu 100% identisch mit der in SEQ ID NO:1 angegebenen Aminosäuresequenz.

In weiteren verschiedenen Ausführungsformen der Erfindung ist die Lipase ein frei vorliegendes Enzym. Dies bedeutet, dass die Lipase mit allen Komponenten eines Mittels direkt interagieren kann und, falls es sich bei dem Mittel um ein flüssiges Mittel handelt, dass die Lipase direkt mit dem Lösungsmittel des erfindungsgemäßen Mittel (z.B. Wasser) in Kontakt steht. In anderen Ausführungsformen kann die erfindungsgemäße Lipase in einem Mittel einen Komplex mit anderen Molekülen bilden oder eine "Umhüllung" umfassen. Hierbei kann ein einzelnes oder mehrere Lipase-Moleküle durch eine sie umgebende Struktur von den anderen Bestandteilen eines Mittels getrennt sein. Eine solche trennende Struktur schließt beispielsweise, ohne darauf beschränkt zu sein, Vesikel, wie etwa eine Micelle oder ein Liposom, ein. Die umgebende Struktur kann aber auch ein Viruspartikel, eine bakterielle Zelle oder eine eukaryotische Zelle sein. In verschiedenen Ausführungsformen kann die erfindungsgemäße Lipase in Zellen von *Alicyclobacillus acidocaldarius,* die diese Lipase exprimiert, oder in Zellkulturüberständen solcher Zellen enthalten sein.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

In einer weiteren Ausführungsform der Erfindung ist die Lipase dadurch gekennzeichnet, dass ihre Reinigungsleistung gegenüber derjenigen einer Lipase, die eine Aminosäuresequenz umfasst, die der in SEQ ID NO:1 angegebenen Aminosäuresequenzen entspricht, nicht signifikant verringert ist, d.h. mindestens 70%, 75 %, 80 %, 85 %, 90 %, 95 % der Referenzwaschleistung besitzt. Die Reinigungsleistung kann in einem Waschsystem bestimmt werden, das ein Waschmittel in einer Dosierung zwischen 4,5 und 7,0 Gramm pro Liter Waschflotte sowie die Lipase enthält, wobei die zu vergleichenden Lipasen konzentrationsgleich (bezogen auf aktives Protein) eingesetzt sind und die Reinigungsleistung gegenüber einer Anschmutzung auf Baumwolle bestimmt wird durch Messung des Reinigungsgrades der gewaschenen Textilien. Beispielsweise kann der Waschvorgang für 30 Minuten bei einer Temperatur von 40°C erfolgen und das Wasser eine Wasserhärte zwischen 5 und 25°, bevorzugt 10 und 20°, bevorzugter 13 und 17° und ferner bevorzugt 15,5 und 16,5° (deutsche Härte) aufweisen. Die Konzentration der Lipase in dem für dieses Waschsystem bestimmten Waschmittel beträgt von 0,001-1 Gew.-%, vorzugsweise von 0,001-0,1 Gew.-%, und noch bevorzugter von 0,01 bis 0,06 Gew.-%, bezogen auf aktives, gereinigtes Protein.

Eine bevorzugte Waschmittelformulierung für ein solches Waschsystem ist wie folgt zusammengesetzt (alle Angaben in Gewichts-Prozent): 5-7% anionische Tenside, 3-5% nicht-ionische Tenside, 0-1% Builder (Zitronensäure und Phosphonate), 0-1% Natronlauge, 0-1% Palmkernölfesttsäure, 0-1% Glycerin, 1-3% Natriumchlorid, 0-1% Borsäure, 0-1% weitere Zusätze (Konservierungsmittel, Entschäumer, opt. Aufheller, Farbstoff, Parfüm), Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird in einem pH-Wertebereich zwischen pH 7,5 und pH 10,5, bevorzugt zwischen pH 7,5 und pH 9 gewaschen.

Im Rahmen der Erfindung kann die Bestimmung der Reinigungsleistung bei 40°C unter Verwendung eines flüssigen Waschmittels wie vorstehend angegeben erfolgen, wobei der Waschvorgang vorzugsweise für 30 Minuten erfolgt.

Der Weißheitsgrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Lipase kann sichergestellt werden, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Die Lipaseaktivität wird in fachüblicher Weise bestimmt, und zwar vorzugsweise wie beschrieben in Bruno Stellmach, "Bestimmungsmethoden Enzyme für Pharmazie, Lebensmittelchemie, Technik, Biochemie, Biologie, Medizin" (Steinkopff Verlag Darmstadt, 1988, S. 172ff). Hierbei werden Lipasehaltige Proben zu einer Olivenölemulsion in emulgatorhaltigem Wasser gegeben und bei 30°C und pH 9,0 inkubiert. Dabei werden Fettsäuren freigesetzt. Diese werden mit einem Autotitrator über 20min. laufend mit 0,01 N Natronlauge titriert, so dass der pH-Wert konstant bleibt ("pH-stat-Titration"). Anhand des Natronlauge-Verbrauchs erfolgt mittels Bezug auf eine Referenzlipaseprobe die Bestimmung der Lipaseaktivität.

Ein alternativer Test zur Feststellung der lipolytischen Aktivität der erfindungsgemäßen Lipasen ist ein optisches Messverfahren, bevorzugt ein photometrisches Verfahren. Der hierfür geeignete Test umfasst die Lipase-abhängige Spaltung eines para-Nitrophenol-Substrates (p-NP-Substrat). Dieses wird durch die Lipase in para-Nitrophenolat und das Substrat (z.B. C2-, C4-, C6-, C8-, C10-, C12-, C14- oder C16-Substrat) gespalten. Die Anwesenheit von para-Nitrophenolat kann unter Verwendung eines Photometers, z.B. des Tecan Sunrise Geräts und der XFLUOR Software, bei 405 nm ermittelt werden und ermöglicht somit einen Rückschluss auf die enzymatische Aktivität der Lipase.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. M. Bender et al., J. Am. Chem. Soc. 88, 24 (1966), S. 5890-5913) erfolgen.

Proteine können über die Reaktion mit einem Antiserum oder einem bestimmten Antikörper zu Gruppen immunologisch verwandter Proteine zusammengefasst werden. Die Angehörigen einer solchen Gruppe zeichnen sich dadurch aus, dass sie dieselbe, von einem Antikörper erkannte antigene Determinante aufweisen. Sie sind daher einander strukturell so ähnlich, dass sie von einem Antiserum oder bestimmten Antikörpern erkannt werden. Einen weiteren Erfindungsgegenstand bilden daher Lipasen, die dadurch gekennzeichnet sind, dass sie mindestens eine und zunehmend bevorzugt zwei, drei oder vier übereinstimmende antigene Determinanten mit einer erfindungsgemäßen Lipase aufweisen. Solche Lipasen sind auf Grund ihrer immunologischen Übereinstimmungen den erfindungsgemäßen Lipasen strukturell so ähnlich, dass auch von einer gleichartigen Funktion auszugehen ist.

Erfindungsgemäße Lipasen können im Vergleich zu der in SEQ ID NO:1 beschriebenen Lipase weitere Aminosäureveränderungen, insbesondere Aminosäure-Substitutionen, -Insertionen oder-Deletionen, aufweisen. Solche Lipasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Lipasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Lipase noch weiter erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Lipase, zum Beispiel hinsichtlich ihrer Aktivität und/oder ihrer Toleranz in Bezug auf das Substratspektrum, kann daher im Rahmen der Erfindung zusätzlich weiterentwickelt sein.

Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt N15Q die Substitution von Asparagin an Position 15 durch Glutamin, N15NA die Insertion von Alanin nach der Aminosäure Asparagin an Position 15 und N15* oder ΔN15 die Deletion von Asparagin an Position 15. Diese Nomenklatur ist dem Fachmann auf dem Gebiet der Enzymtechnologie bekannt.

Ein weiterer Gegenstand der Erfindung ist daher eine Lipase, die dadurch gekennzeichnet ist, dass sie aus einer Lipase wie vorstehend beschrieben als Ausgangsmolekül erhältlich ist durch ein- oder mehrfache konservative Aminosäuresubstitution. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Lipase dadurch gekennzeichnet, dass sie aus einer erfindungsgemäßen Lipase als Ausgangsmolekül erhältlich ist durch Fragmentierung, Deletions-, Insertions-oder Substitutionsmutagenese und eine Aminosäuresequenz umfasst, die über eine Länge von mindestens 220, 230, 240, 250, 260, 270, 280, 290, 300 oder 310 zusammenhängenden Aminosäuren mit dem Ausgangsmolekül übereinstimmt.

So ist es beispielsweise möglich, an den Termini oder in den Loops des Enzyms einzelne Aminosäuren zu deletieren, ohne dass dadurch die hydrolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre hydrolytische Aktivität, d.h. ihre hydrolytische Aktivität entspricht mindestens derjenigen des Ausgangsenzyms, d.h. in einer bevorzugten Ausführungsform beträgt die hydrolytische Aktivität mindestens 80, vorzugsweise mindestens 90 % der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Die weiteren Aminosäurepositionen werden hierbei durch ein Alignment der Aminosäuresequenz einer erfindungsgemäßen Lipase mit der Aminosäuresequenz der Lipase aus *Alicyclobacillus acidocaldarius,* wie sie in SEQ ID NO:1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Lipase eine höhere Zahl von Aminosäurenresten umfasst als die Lipase aus *Alicyclobacillus acidocaldarius* gemäß SEQ ID NO:1. Ausgehend von den genannten Positionen in der Aminosäuresequenz der Lipase aus *Alicyclobacillus acidocaldarius* sind die Veränderungspositionen in einer erfindungsgemäßen Lipase diejenigen, die eben diesen Positionen in einem Alignment zugeordnet sind.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Lipasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Lipase aus *Alicyclobacillus acidocaldarius* gemäß SEQ ID NO:1 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Lipase-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Lipase anwendbar. Demnach umfasst ein erfindungsgemäßes Verfahren ferner einen oder mehrere der folgenden Verfahrensschritte:
a) Einbringen einer ein- oder mehrfachen konservativen Aminosäuresubstitution in eine AusgangsLipase gemäß SEQ ID NO:1;
b) Veränderung der Aminosäuresequenz durch Fragmentierung, Deletions-, Insertions- oder Substitutionsmutagenese derart, dass die Lipase eine Aminosäuresequenz umfasst, die über eine Länge von mindestens 210, 220, 230, 240, 250, 260, 270, 280, 290, 300, 301, 302, 303, 304, 305, 306, 307, 308 oder 309 zusammenhängenden Aminosäuren mit dem Ausgangsmolekül übereinstimmt.

Sämtliche Ausführungen gelten auch für die erfindungsgemäßen Verfahren.

In weiteren Ausgestaltungen der Erfindung ist die Lipase beziehungsweise die mit einem erfindungsgemäßen Verfahren hergestellte Lipase noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,8%, 99,0%, 99,2%, 99,4%, 99,6% oder 99,8% identisch zu der in SEQ ID NO:1 angegebenen Aminosäuresequenz über deren Gesamtlänge.

Ein weiterer Gegenstand der Erfindung ist eine zuvor beschriebene Lipase, die zusätzlich stabilisiert ist, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Möglichkeiten der Stabilisierung sind beispielsweise:
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Lipase wie vorstehend beschrieben, die dadurch gekennzeichnet ist, dass sie mindestens eine chemische Modifikation aufweist. Eine Lipase mit einer solchen Veränderung wird als Derivat bezeichnet, d.h. die Lipase ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Lipasen mit spezifischen Inhibitoren ist diesbezüglich möglich.

Betreffend alle vorstehend beschriebenen Lipasen beziehungsweise Lipasevarianten und/oder Derivate sind im Rahmen der vorliegenden Erfindung diejenigen besonders bevorzugt, deren katalytische Aktivität und/oder deren Substrattoleranz derjenigen der Lipase gemäß SEQ ID NO:1 entspricht, wobei die katalytische Aktivität und die Substrattoleranz wie vorstehend beschrieben bestimmt werden.

Ein weiterer Gegenstand der Erfindung ist eine Nukleinsäure, die für eine erfindungsgemäße Lipase codiert, sowie ein Vektor enthaltend eine solche Nukleinsäure, insbesondere ein Klonierungsvektor oder ein Expressionsvektor. In bevorzugten Ausführungsformen ist die Nukleinsäure eine Nukleinsäure gemäß SEQ ID NO:2. Entsprechend ist ein besonders bevorzugter erfindungsgemäßer Vektor ein Vektor, der eine Nukleinsäure gemäß SEQ ID NO:2 umfasst.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Lipasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Lipase beinhaltet, insbesondere eine, die die Lipase in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Lipasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Lipase funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Ac*tinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung *Alicyclobacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebakterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.*

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie *Saccharomyces* oder *Kluyveromy*ces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Proteine oder Varianten eignen. In bevorzugten Ausführungsformen der Erfindung ist die Wirtszelle eine Basidiomyceten-Zelle. In ferner bevorzugten Ausführungsformen ist die Wirtszelle eine *Alicyclobacillus acidocaldarius* Zelle.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Lipasen herzustellen. Ein weiterer Gegenstand der Erfindung ist daher ein Verfahren zur Herstellung einer Lipase umfassend
a) Kultivieren einer erfindungsgemäßen Wirtszelle, und
b) Isolieren der Lipase aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Lipase. Alle Fermentationsverfahren, die auf einem entsprechenden Verfahren zur Herstellung einer erfindungsgemäßen Lipase beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Lipase erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Lipase kann aus dem Fermentationsmedium geerntet werden. Ein solches Fermentationsverfahren ist gegenüber einer Isolation der Lipase aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Lipase in das Fermentationsmedium sezernieren. Ohne Sekretion kann alternativ die Isolation der Lipase aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Lipasen herzustellen.

Ein weiterer Gegenstand der Erfindung ist ein Mittel, das dadurch gekennzeichnet ist, dass es eine erfindungsgemäße Lipase wie vorstehend beschrieben enthält. Bevorzugt ist das Mittel als ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Lipase alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Lipase mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Lipase mit einem Tensid und/oder einem Builder (Gerüststoff) und/oder einer Persauerstoffverbindung und/oder einem Bleichaktivator kann ein solcher Synergismus erreicht werden.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die Lipase vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. Darüber hinaus kann das erfindungsgemäße Mittel die Lipase vorteilhafterweise in einer Menge von 0,00005-15 Gew.-% bezogen auf das aktive Enzym, vorzugsweise von 0,0001-5 Gew.-% und besonders bevorzugt von 0,001-1 Gew.-% enthalten. Ferner kann die in dem Mittel enthaltene Lipase, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Lipase mit einer bei Raumtemperatur oder bei Abwesenheit von Wasser für die Lipase undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) in gelförmiger oder dosierbeutelförmiger (Pouches) Form vorliegt, und/oder
(d) als Einkomponentensystem vorliegt, oder
(e) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine Lipase enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder andere - von den erfindungsgemäßen Lipasen unterscheidbare - Lipasen, sowie deren Gemische. Weitere Enzyme sind in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein.

Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Lipase und einem weiteren Enzym eines erfindungsgemäßen Mittels, darunter insbesondere zwischen der genannten Lipase und einer Amylase und/oder einer Protease und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Die Enzyme können auch in wasserlösliche Filme eingebracht werden. Ein derartiger Film ermöglicht die Freisetzung der Enzyme nach Kontakt mit Wasser. Wie hier verwendet, bezieht sich "wasserlöslich" auf eine Filmstruktur, die vorzugsweise vollständig wasserlöslich ist. Jedoch sind auch Filme, die im Wesentlichen wasserlöslich sind, aber relativ kleine Mengen eines Materials in der Filmstruktur aufweisen, welches nicht wasserlöslich ist; Folien mit Materialien, die nur bei relativ hohen Wassertemperaturen oder nur unter eingeschränkten pH-Bedingungen wasserlöslich sind; und Folien, die eine relativ dünne Schicht aus wasserunlöslichem Material einschließen, alle in der Bezeichnung "wasserlöslich" mit eingeschlossen. Bevorzugt besteht ein solcher Film aus (vollständig oder teilweise hydrolysiertem) Polyvinylalkohol (PVA). Der Film kann aber auch ausschließlich oder zusätzlich zum PVA enthalten Säure/Acrylat-Copolymere, vorzugsweise Methacrylsäure/Ethylacrylat-Copolymer, wie das von Belland als GBC 2580 und 2600 erhältliche; Styrol-Maleinsäureanhydrid-Copolymer (SMA) (verfügbar als Scripset (Handelsname) von Monsanto); Ethylen-Acrylsäure-Copolymer (EAA) oder durch Metallsalz neutralisiertes Ethylen-Methacrylsäure-Copolymer (EMAA), bekannt als lonomer (verfügbar von du Pont), bei welchem der Säuregehalt von EAA oder EMAA mindestens etwa 20 Mol-% beträgt; Polyether-Blockamid- Copolymer; Polyhydroxyvalerinsäure (verfügbar als Biopol (Handelsname)-Harze von Imperial Chemical Industries); Polyethylenoxid; wasserlöslichen Polyester oder Copolyester; Polyethyloxazolin (PEOX 200 von Dow); und wasserlösliches Polyurethan ein.

Weiterhin ist es möglich, zwei oder mehrere Enzyme zusammen zu konfektionieren, so dass ein einzelnes Granulat mehrere Enzymaktivitäten aufweist.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Lipase katalytisch aktiv wird, insbesondere derart, dass die Lipase in einer Menge von 40µg bis 4g, vorzugsweise von 50µg bis 3g, besonders bevorzugt von 100µg bis 2g und ganz besonders bevorzugt von 200µg bis 1g eingesetzt wird.

In verschieden Ausführungsformen zeichnet sich das oben beschriebene Verfahren dadurch aus, dass die Lipase bei einer Temperatur von 0-100°C, bevorzugt 0-60°C, weiter bevorzugt 20-45°C und am meisten bevorzugt bei 40°C eingesetzt wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Lipase bereichert werden und stellen dann Ausführungsformen der vorliegenden Erfindung dar. Alle Sachverhalte, Gegenstände und Ausführungsformen, die für erfindungsgemäße Lipasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Lipasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass gewünschtenfalls als einzige reinigungsaktive Komponente eine erfindungsgemäße Lipase mit der Anschmutzung in Kontakt gebracht wird, bevorzugt in einer Pufferlösung oder in Wasser. Dies stellt eine weitere Ausführungsform dieses Erfindungsgegenstandes dar.

In einem weiteren Aspekt bezieht sich die vorliegende Erfindung auf die Verwendung einer erfindungsgemäßen Lipase oder einer nach einem erfindungsgemäßen Verfahren erhältliche Lipase in einem Wasch- oder Reinigungsmittel zur Entfernung von lipidhaltigen Anschmutzungen. Alle Sachverhalte, Gegenstände und Ausführungsformen, die für erfindungsgemäße Lipase und sie enthaltende Mittel beschrieben sind, sind auch auf diesen Erfindungsgegenstand anwendbar.

### Beispiele

### Beispiel 1:

### Isolierung des Lipase-Gens

In einem aktivitätsbasierten Screening wurden sieben Metagenomdatenbanken und insgesamt ca. 50.000 Klone durchmustert. Dabei konnten 13 Klone isoliert werden, die Aktivität auf mit Tributyrin versetzten LB-Agar-Platten aufwiesen. Außerdem wurde die Aktivität von 26 "Falsch-Positiven" nach mehrmaligen Überimpfungs- und Vereinzelungsschritten widerlegt. Die Plasmid-DNA konnte aus den 13 möglichen positiven Klonen isoliert werden und mittels enzymatischem Verdau wurde die ungefähre Insertionsgröße bestimmt. Die isolierte Plasmid-DNA wurde schrittweise sequenziert und ergab 10 verschiedene Open Reading Frames, in welchem die aufgeführte Ziellipase identifiziert wurde.

### Rekombinante Expression

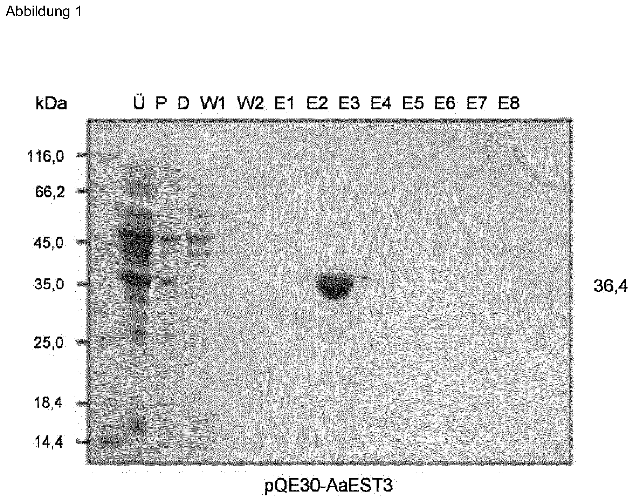

Zur rekombinanten Expression der Ziellipase wurde das Gen in ein pQE30 Expressionsplasmid kloniert und im Stamm E.coli M15[pREP4] produziert. Für die Reinigung mittels Affinitätschromatographie wurden 500 ml-Kultur angezogen. Die Induktion wurde mit 0,5 mM IPTG eingeleitet und die Anzucht erfolgte bei 37°C für 4h. Die Ziellipase konnte mittels Ni-NTA-Affinitätschromatographie bereits in den ersten Elutionsschritten von der Säulenmatrix eluiert werden (Abbildung 1).

### Bestimmung von Substrat-, Temperatur- und pH-Profil

Zur Bestimmung der verschiedenen Aktivitäten muss stets eine Verdünnung gewählt werden in der es zu keiner Substrat-Sättigung während der Messung kommt. Für alle Messungen erfolgte zunächst eine Dialyse der aus der Affinitätsreinigung gewonnenen Probe gegen Tris-Puffer (50 mM, pH 8,0).

Folgende Reaktionslösungen werden für die Messungen benötigt:
Lösung A: 9 ml Puffer + 100 mg Gum arabicum
Lösung B: Puffer
Lösung C: 2 mM Substrat (= p-NP-Ester)

Zur Messung der Aktivität werden zunächst 810 µl von Reaktionslösung A, 90 µl von Lösung B, 90 µl von Lösung C zusammenpipettiert, gut gevortext und für 2-5 min in einem Heizblock bei der entsprechend relevanten Temperatur vorinkubiert. Anschließend werden 10 µl Enzym zugegeben, gevortext und über die Dauer von 2 min bei 410 nm die Absorption (OD) aufgezeichnet. Als Blank wird statt 10 µL Enzym nur Puffer eingesetzt.

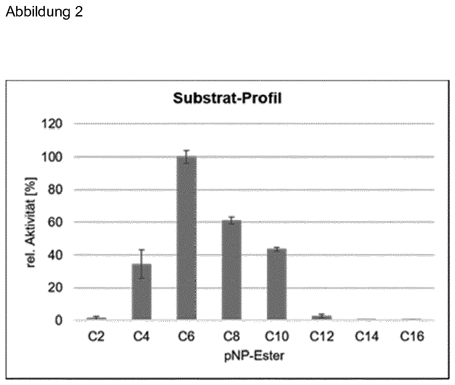

Zur Bestimmung des Substratprofils wurden die Aktivitäten in Tris-Puffer (50 mM, pH 8,0) bei einer Temperatur von 60 °C aufgezeichnet. Dabei wurde die Aktivität gegenüber C2, C4, C6, C8, C10, C12, C14 und C16 ermittelt (Abbildung 2).

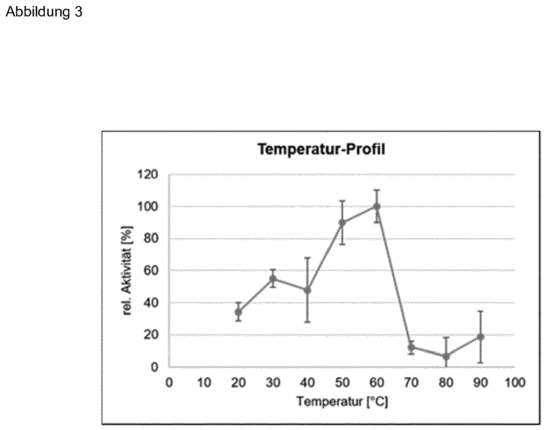

Zur Bestimmung des Temperatur-Profils erfolgten Messungen in Tris-Puffer (50 mM, pH 8,0). Also Substrat wurde p-NP-C6 verwendet (Abbildung 3).

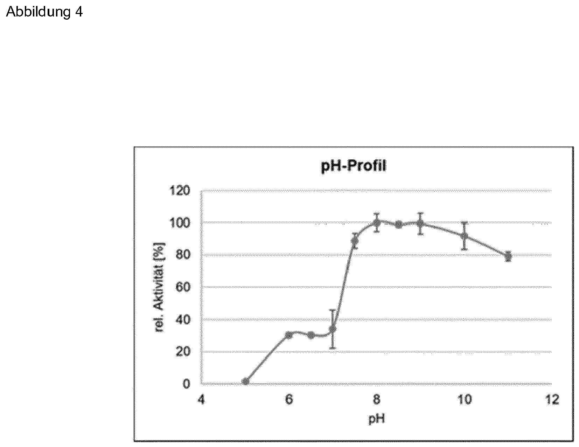

Zur Bestimmung des pH Profils erfolgten die Aktivitätsmessungen in Britton-Robinson-Puffer (= Universal-Puffer) (4,59 ml H3PO4 (85%), 4,56 ml Essigsäure (98%), 4,99 g Borsäure, 120 mM, pH 5-11). Hierzu wurde für Lösung A dementsprechende Mengen an *Gum arabicum* hinzugefügt. Die Reaktionstemperatur betrug 40 °C, als Substrat wurde p-NP-C4 verwendet (Abbildung 4).

Wie aus der Charakterisierung der erfindungsgemäßen Lipase hervorgeht (Abbildungen 2-4), weist sie Aktivitäten gegenüber Fettsäuren mit einer Kettenlänge von 4-10 auf. Weiterhin liegt das Temperaturoptimum im Bereich von 50-60°C, wobei bei 20-40°C noch ca. 60% Restaktivität detektierbar sind. Zusätzlich ist wurde ein breites pH Optimum im Bereich von pH 7-10 bestimmt. Somit eignet sich dieser neue Lipase-Wildtyp gut für den Einsatz in Wasch- und Reinigungsmitteln.

### Waschtest im Launder-o-meter

Zur Überprüfung der Waschleistung wurde aufgereinigter Überstand aus *E.coli,* in welchem die beschriebene Lipase exprimiert wurde, in einem Launder-o-meter Waschversuch eingesetzt. Die Waschversuche wurden in 3-fach Bestimmung durchgeführt.

### Bedingungen:

Waschtemperatur: 40°C
Wasserhärte: 16°dH Wasser
Dauer: 30 min
Waschvolumen: 200mL
Flottenverhältnis: 1:12 (zusammengesetzt aus Steifen Testlappen + Füll-Läppchen mit 10 Stahlkugeln)
Waschmitteldosierung: 1,9 g/L
Enzymkonzentration: 0,008% aktive Lipase

### Anschmutzungen:

**Tabelle 1:**

| | |
|---|---|
| Wfk 10D | pigment/sebum (CO) |
| Wfk 20D | pigment/sebum (PES/CO) |
| Wfk 20C | pigment/lanolin (PES/CO) |
| CFT C09 | pigment/oil cotton |
| | Biskin |
| | Schweineschmalz |

### Waschmittelformulierung:

**Tabelle 2:**

| **Inhaltsstoffe** | **Rezeptur 1** |
|---|---|
| | |
| Aniontensid | 5-7% |
| Niotensid | 3-5% |
| Builder (Zitronensäure und Phosphonate) | 0-1% |
| Natronlauge | 0-1% |
| Palmkernölfettsäure | 0-1% |
| Glycerin | 0-1% |
| Natriumchlorid | 1-3% |
| Borsäure | 0-1% |
| Propylenglykollaurat | - |
| Weitere Zusätze (Konservierungsmittel, Entschäumer, opt. Aufheller, Farbstoff, Parfüm) | 0-1% |
| Wasser | Rest |

Zur Auswertung wurden die Helligkeitswerte der verwendeten Anschmutzungen mithilfe des Farbmessgerätes Minolta CR400-1 bestimmt. Lichtart: D65, 2°. Auswertung: HSD (Tukey) für ungleiche Stichproben-Umfänge

### Ergebnisse:

**Tabelle 3:**

| | Rezeptur 1 ohne Lipase | Rezeptur 1 mit erfindungsgemäßer Lipase |
|---|---|---|
| pigment/sebum (CO) | 49,0 | 50,7 |
| pigment/sebum (PES/CO) | 51,2 | 53,0 |
| pigment/lanolin (PES/CO) | 41,6 | 42,1 |
| pigment/oil cotton | 52,8 | 53,5 |
| Biskin | 34,5 | 36,3 |
| Schweineschmalz | 37,9 | 47,0 |

Es wird deutlich, dass die Lipase auf verschiedenen fetthaltigen Anschmutzungen einen Vorteil zeigt. Von einer signifikanten Leistungsverbesserung spricht man schon ab 1 Einheit, in diesem Versuch wurden beispielsweise auf Schweineschmalz 9 Einheiten Verbesserung erzielt. Als Negativkontrolle wurde der abgekochte, aufgereinigte Überstand aus dem Produktionsorganismus *E.coli* mitgewaschen (99°C für 30 min), der keinerlei Waschleistung zeigt.

## Patentansprüche

1. Lipase umfassend eine Aminosäuresequenz, die mindestens 70 % Sequenzidentität mit der in SEQ ID NO:1 angegebenen Aminosäuresequenz über deren Gesamtlänge aufweist.

2. Lipase, **dadurch gekennzeichnet, dass**
(a) sie aus einer Lipase nach Anspruch 1 als Ausgangsmolekül erhältlich ist durch ein- oder mehrfache konservative Aminosäuresubstitution; und/oder
(b) sie aus einer Lipase nach Anspruch 1 als Ausgangsmolekül erhältlich ist durch Fragmentierung, Deletions-, Insertions- oder Substitutionsmutagenese und eine Aminosäuresequenz umfasst, die über eine Länge von mindestens 220, 230, 240, 250, 260, 270, 280, 290, 300, 301, 302, 303, 304, 305, 306, 307, 308 oder 309 zusammenhängenden Aminosäuren mit dem Ausgangsmolekül übereinstimmt.

3. Verfahren zur Herstellung einer Lipase nach Anspruch 1 oder 2 umfassend das Bereitstellen einer Ausgangslipase, die mindestens 70 % Sequenzidentität zu der in SEQ ID NO:1 angegebenen Aminosäuresequenz über deren Gesamtlänge aufweist.

4. Verfahren nach Anspruch 3, ferner umfassend einen oder beide der folgenden Verfahrensschritte:
(a) Einbringen einer ein- oder mehrfachen konservativen Aminosäuresubstitution;
(b) Veränderung der Aminosäuresequenz durch Fragmentierung, Deletions-, Insertions- oder Substitutionsmutagenese derart, dass die Lipase eine Aminosäuresequenz umfasst, die über eine Länge von mindestens 220, 230, 240, 250, 260, 270, 280, 290, 300, 301, 302, 303, 304, 305, 306, 307, 308 oder 309 zusammenhängenden Aminosäuren mit dem Ausgangsmolekül übereinstimmt.

5. Nukleinsäure codierend für eine Lipase nach einem der Ansprüche 1 oder 2 oder codierend für eine nach einem Verfahren der Ansprüche 3 oder 4 erhaltene Lipase.

6. Vektor enthaltend eine Nukleinsäure nach Anspruch 5, insbesondere ein Klonierungsvektor oder ein Expressionsvektor.

7. Nicht menschliche Wirtszelle, die eine Nukleinsäure nach Anspruch 5 oder einen Vektor nach Anspruch 6 beinhaltet, oder die eine Lipase nach einem der Ansprüche 1 oder 2 beinhaltet, oder die eine nach einem Verfahren der Ansprüche 3 oder 4 erhaltene Lipase beinhaltet, insbesondere eine, die die Lipase in das die Wirtszelle umgebende Medium sezerniert.

8. Verfahren zur Herstellung einer Lipase umfassend
a) Kultivieren einer Wirtszelle gemäß Anspruch 7; und
b) Isolieren der Lipase aus dem Kulturmedium oder aus der Wirtszelle.

9. Mittel, insbesondere ein Wasch- oder Reinigungsmittel, **dadurch gekennzeichnet, dass** es mindestens eine Lipase nach einem der Ansprüche 1 oder 2 oder eine nach einem Verfahren der Ansprüche 3 oder 4 erhaltene Lipase enthält, insbesondere ist die Lipase in einer Menge bis zu 1 Gew.-% bezogen auf aktives Protein enthalten.

10. Verfahren zur Reinigung von Textilien oder harten Oberflächen, **dadurch gekennzeichnet, dass** in mindestens einem Verfahrensschritt ein Mittel nach Anspruch 9 angewendet wird, oder dass in mindestens einem Verfahrensschritt eine Lipase nach einem der Ansprüche 1 oder 2 oder eine nach einem Verfahren der Ansprüche 3 oder 4 erhaltene Lipase katalytisch aktiv wird.

11. Verwendung einer Lipase nach einem der Ansprüche 1 oder 2 oder einer nach einem Verfahren der Ansprüche 3 oder 4 erhältliche Lipase in einem Wasch-oder Reinigungsmittel zur Entfernung von lipidhaltigen Anschmutzungen.


SEQUENCE LISTING

<110>  Henkel AG & Co. KGaA

<120>  Neue Lipase

<130>  PT034041

<160>  2

<170>  PatentIn version 3.5

<210>  1
<211>  310
<212>  PRT
<213>  Alicyclobacillus acidocaldarius

<400>  1

Met Pro Leu Asp Pro Val Ile Gln Gln Val Leu Asp Gln Leu Asn Arg
1               5                   10                  15

Met Pro Ala Pro Asp Tyr Gln His Leu Ser Ala Gln Gln Phe Arg Ser
            20                  25                  30

Gln Gln Ser Leu Phe Pro Pro Val Lys Lys Glu Pro Val Ala Glu Val
        35                  40                  45

Arg Glu Phe Asp Met Asp Leu Pro Gly Arg Thr Leu Lys Val Arg Met
    50                  55                  60

Tyr Arg Pro Glu Gly Val Glu Ala Pro Tyr Pro Ala Leu Val Tyr Tyr
65                  70                  75                  80

His Gly Gly Gly Trp Val Val Gly Asp Leu Glu Thr His Asp Pro Val
                85                  90                  95

Cys Arg Val Leu Ala Lys Asp Gly Arg Ala Val Val Phe Ser Ile Asp
            100                 105                 110

Tyr Arg Leu Ala Pro Glu His Lys Phe Pro Ala Ala Val Glu Asp Ala
        115                 120                 125

Tyr Asp Ala Leu Gln Trp Ile Ala Glu Arg Ala Ala Asp Phe Gln Leu
    130                 135                 140

Asp Pro Ser Arg Ile Ala Val Gly Gly Asp Ser Ala Gly Gly Asn Leu
145                 150                 155                 160

Ala Ala Val Thr Ser Ile Leu Ala Lys Glu Arg Gly Gly Pro Ala Ile
                165                 170                 175

Ala Phe Gln Leu Leu Ile Tyr Pro Ser Thr Gly Tyr Asp Pro Ala His
            180                 185                 190

Pro Pro Ala Ser Ile Glu Glu Asn Ala Glu Gly Tyr Leu Leu Thr Gly
        195                 200                 205

Gly Met Met Leu Trp Phe Arg Asp Gln Tyr Leu Asn Ser Leu Glu Glu
    210                 215                 220

Leu Thr His Pro Trp Phe Ser Pro Val Leu Tyr Pro Asp Leu Ser Gly
225                 230                 235                 240

Leu Pro Pro Ala Tyr Ile Ala Thr Ala Gln Tyr Asp Pro Leu Arg Asp
                245                 250                 255

Val Gly Lys Leu Tyr Ala Glu Ala Leu Gln Lys Ala Gly Val Lys Val
            260                 265                 270

Glu Ile Glu Asn Phe Glu Asp Leu Ile His Gly Phe Ala Gln Phe Tyr
        275                 280                 285

Ser Leu Ser Pro Gly Ala Thr Lys Ala Leu Leu Arg Ile Ala Glu Lys
    290                 295                 300

Leu Arg Glu Ala Leu Ala
305                 310

<210>  2
<211>  933
<212>  DNA
<213>  Alicyclobacillus acidocaldarius

<400>  2
atgccgctcg atcccgtcat tcaacaggtg ctcgatcaac tcaatcgcat gcccgcccct      60

gattaccaac atctctctgc ccagcaattt cgctcccaac agtcgctgtt tccacccgtc     120

aaaaaggagc ccgtcgccga ggtccgagag tttgacatgg accttcccgg ccgcacgctc     180

aaggtccgca tgtaccgccc ggagggcgtc gaagcgccgt acccagcact cgtgtattat     240

cacggcggcg gttgggtcgt cggagacctc gagacgcacg atcccgtctg ccgcgtcctc     300

gcgaaggacg gccgcgctgt cgtcttctcc atcgactacc gccttgcgcc cgaacacaag     360

tttcctgccg ctgtggaaga cgcttacgac gcgcttcagt ggattgcgga gcgcgcggcg     420

gactttcagc tcgatccgtc ccgcatcgcc gtcgggggcg acagcgctgg aggcaacctg     480

gcagctgtaa cgagcatcct ggccaaggag cgcggcggcc cggccatcgc gttccagctg     540

ctcatctacc cttccacggg gtacgatccg gcccatcctc ccgcatctat cgaggaaaat     600

gcggaaggct atctcctgac cggcggcatg atgctctggt tccgggatca atacctgaac     660

agcctggagg aactcacgca tccgtggttc tcacccgtcc tctaccccga tttgagcggc 720

ttgccgccgg cgtacatcgc aacggcgcag tacgatccgc tgcgcgacgt cggcaagctt 780

tacgcggaag cgctgcaaaa ggcgggcgtc aaggtcgaga ttgagaactt tgaagatctg 840

atccacggat tcgctcagtt ttacagcctc tcgcccggcg cgacgaaggc actcctccgc 900

attgcggaaa aacttcgaga ggcgctggcg tga 933